(19) Europäisches Patentamt
European Patent Office
Office européen des brevets

(11) Publication number: **0 250 614 A1**

(12) # EUROPEAN PATENT APPLICATION

(21) Application number: **86108541.3**

(22) Date of filing: **23.06.86**

(51) Int. Cl.4: **C12N 15/00**, C12N 1/20, A61K 39/112, A61K 39/108, //(C12N1/20,C12R1:01,1:42,1:1-85)

The applicant has filed a statement in accordance with Rule 28 (4) EPC (issue of a sample only to an expert). Accession numbers of the deposits: DSM 3762 - DSM 3760 - DSM 3761.

The title of the invention has been amended (Guidelines for Examination in the EPO, A-III, 7.3).

(43) Date of publication of application:
**07.01.88 Bulletin 88/01**

(84) Designated Contracting States:
**DE**

(71) Applicant: **Timmis, Kenneth N. Prof.**
**c/o Département de Biochimie Médicale
Centre Médical Universitaire 9, avenue de Champel
CH-1211 Genève 4(CH)**

(72) Inventor: **Timmis, Kenneth N. Prof.**
**c/o Département de Biochimie Médicale
Centre Médical Universitaire 9, avenue de Champel
CH-1211 Genève 4(CH)**

(74) Representative: **Vossius & Partner
Siebertstrasse 4 P.O. Box 86 07 67
D-8000 München 86(DE)**

(54) **DNA fragments encoding the chromosomal nucleotide sugar synthetases and glycosyl transferases.**

(57) DNA fragments encoding the chromosomal nucleotide sugar synthetases and glycosyltransferases involved in the biosynthesis of the O-antigen of Shigella dysenteriae, recombinant DNA molecules containing said fragments, host organisms containing said recombinant DNA molecules, vaccines for preventing bacillary dysentery and method for preventing bacillary dysentery.

The present invention relates to DNA fragments encoding the chromosomal nucelotide sugar synthetases and glycosyl-transferases involved in the biosynthesis of the O-antigen of Shigella dysenteriae and to recombinant DNA molecules containing said fragments. The recombinant DNA molecules of the present invention are introduced in host organisms and used for the production of vaccines for preventing bacillary dysentery.



Xerox Copy Centre

# DNA fragments encoding the chromosomal nucleotide sugar synthetases and glycosyltransferases involved in the biosynthesis of the O-antigen of Shigella dysenteriae, recombinant DNA molecules containing said fragments, host organisms containing said recombinant DNA molecules, vaccines for preventing bacillary dysentery and method for preventing bacillary dysentery

The invention relates to DNA fragments encoding the chromosomal nucleotide sugar synthetases and glycosyltransferases involved in the biosynthesis of the O-antigen of Shigella dysenteriae 1. Furthermore the invention relates to a biotechnological process for preparing living or killed S. dysenteriae 1 O-antigen-producing bacteria and to a vaccine containing such bacteria.

Shigella and some enteroinvasive strains of Escherichia coli cause bacillary dysentery, an acute but generally self-limiting ulcerative colitis. Although the incidence of bacillary dysentery in developed countries is low, it is much higher in developing countries, particularly in certain countries of Africa, Latin America, Asia, and in China, and constitutes an important component of diarrhoeal disease (more precisely of "bloody diarrhoea ") in such regions. Moreover, in conditions where nutrition is suboptimal and other infections are prevalent, dysentery is not infrequently life-threatening, due in part to an increased severity of the disease and in part to the development of complications such as leukaemoid reactions, haemolytic-uraemic syndrome, sepsis and seizures, which tend to be characterized by high fatality rates, particularly in infants. Shigella dysenteriae 1 provokes the most severe form of dysentery and is the most frequent cause of complications. It is currently responsible for serious dysentery epidemics in a number of developing countries and represents a public health problem of considerable magnitude. The fact that many isolates exhibit multiple antibiotic resistance complicates the clinical management of dysentery infections. The development of an efficacious vaccine against S. dysenteriae 1 therefore represents a particularly urgent objective.

The lipopolysaccharide (LPS) layer of gram negative bacteria constitutes a major component of the cell surface and an important virulence factor of pathogenic strains. The lipopolysaccharide molecule consists of three structural components

1. Lipid A, which is the innermost portion of the molecule with regard to the cell surface;
2. an oligosaccharide core; and
3. the O-specific polysaccharide, which consists of a variable number of identical oligosaccharide repeat units and which extends away from the cell into the surrounding medium.

It is the O-specific polysaccharide (O-antigen or somatic antigen)-portion of LPS that forms an anti-phagocytic capsule-like structure around the cell and that provides antigenic specificity typical of the O-serogroup of the producing organism.

The following structure of the S. dysenteriae 1 O-antigen has been determined (Dmitriev et al., Enr. J.Biochem. 66(1976), 559-566):

```
 .3              1.3              1.2                1.3                          1
---> rhamnose ---> rhamnose ---> galactose ---> N-acetylglucosamine -
 α  30            α                α                  α
```

but neither the orientation of the sugars in the repeat unit nor the first sugar of the repeat unit have been determined.

Previous studies indicate that an efficacious vaccine is most likely to be a live vaccine for enteral administration which stimulates mucosal immunity to the O-specific lipopolysaccharide (LPS) of shigellae. Thus far, two different types of attenuated Shigella vaccines have been develped, namely a spontaneous mutant that is no longer able to invade the intestinal mucosa, and a Shigella - E.coli hybrid that is able to invade the intestinal mucosa, but that has a reduced ability to multiply in the tissues. Protection against challenge is associated with expression of the Shigella O-antigen by the vaccine strain. Neither type of vaccine was entirely satisfactory due to vaccine instability, residual virulence, or the need for multiple vaccinations.

Until recently, efforts to construct a hybrid vaccine strain expressing the S. dysenteriae 1 O-antigen were unsuccessful. The reason for this became apparent when Watanabe and Timmis, 1984, Infect. Immun. 43, 391, showed that a small 9 kb plasmid, designated pHW400, carried by S. dysenteriae 1 strains, contains a determinant, designated rfp, for O-antigen biosynthesis. This determinant was subsequently localized by transposon mutagenesis to a 2 kb region of the plasmid and its product identified as a 41 000 Dalton polypeptide. Transformation of E. coli with this plasmid showed that it is essential but not sufficient

for production of the O-antigen LPS. Thus, the information for the biosynthesis of the O-antigen is distributed over two distinct genetic elements, the 9 kb plasmid and the bacterial chromosome. Subsequently it was shown that introduction into E. coli K-12 of this plasmid, plus a region of the S. dysenteriae 1 chromosome cotransducible with the his (histidine biosynthesis) gene cluster, and hence the rfb (LPS biosynthesis) gene cluster, resulted in production of S. dysenteriae 1 O-antigen in E.coli. Since the strategy of this latter experiment required the recombination of the Shigella genes into the E. coli K-12 chromosome, it did not permit their isolation for genetic manipulation. In order to be able to introduce readily into different live bacterial antigen delivery systems the ability to produce the S. dysenteriae 1 O-antigen, it is important to localize precisely the chromosomal genes determining O-antigen production, to isolate them by gene cloning, to characterize them, and to combine them in a single recombinant plasmid with the pHW400-plasmid-carried O-antigen determinant.

It is therefore an object of this invention to provide a DNA fragment of the chromosome of Shigella dysenteriae 1 containing the rfb-gene region of S. dysenteriae 1 which encodes the chromosomal nucleotide sugar synthetases and glycosyltransferases involved in the biosynthesis of the O-antigen of S.dysenteriae 1. This novel DNA fragment has a length of 8.9 kb and has the following restriction map:

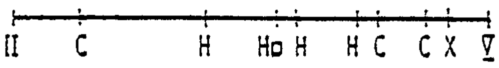


in which the following abbreviations are used:
II, PvuII; C, ClaI; H, Hind III; Hp, HpaI; X, XhoI; V, Eco RV.

It is another object of this invention to provide a DNA fragment hybridizing to the above DNA fragment and encoding the chromosomal nucleotide sugar synthetases and glycosyltransferases involved in the biosynthesis of the O-antigen of S.dysenteriae 1 or a part of the O-antigen, or an antigen that provokes formation of O-antigen cross-reacting antibodies. This fragment may be derived from whatever source obtained including natural, synthetic or semisynthetic source, that is related by mutations, including nucleotide substitutions, nucleotide deletions, nucleotide insertions and inversions of nucleotide stretches to the above DNA fragment.

It is a further object of this invention to provide a recombinant DNA molecule comprising anyone of the above DNA fragments.

It is another object of the present invention to provide a recombinant DNA molecule which contains anyone of the above DNA fragments in combination with a DNA fragment containing the genetic information for the plasmid encoded S. dysenteriae 1 O-antigen biosynthesis function or functions.

In a preferred embodiment of this invention the recombinant DNA molecule containing the genetic information for the plasmid encoded S. dysenteriae 1 O-antigen biosynthesis function(s) is the rfp-gene region of plasmid pHW400 of S. dysenteriae 1.

The recombinant DNA molecule can be used for amplification of its cloned DNA fragments,for production of S. dysenteriae 1 O-antigen in E. coli and other enteric bacteria, and if operatively linked to an expression control sequence, for high level production of O-antigen. The preferred expression cotnrol sequences include the E. coli promoter system, the E. coli lac system, the E. coli ß-lactamase system, the E. coli trp system or the E. coli lipoprotein promoter.

In a more preferred embodiment of this invention the recombinant DNA molecule is plasmid pSS37 deposited with the DSM under the deposition number DSM 3762.

It is another object of this invention to provide as a host organism a gram-negative bacterium which is transformed with any one of the above recombinant DNA molecules.

In a preferred embodiment of this invention the host organism is a species of the genera Shigella, Salmonella or Escherichia.

In a more preferred embodiment of this invention the host organism is the species Escherichia coli K12.

In the most preferred embodiment of this invention the host organism is capable of invading the human intestinal epithelium.

It is another object of this invention to provide a process for preparing S. dysenteriae 1 O-antigen-producing bacteria, characterized by cultivating anyone of the above hosts in a suitable medium and recovering said bacteria, and optionally isolating O-antigen material from said bacteria.

It is still a further object of this invention to provide a vaccine for preventing bacillary dysentery, comprising transformed host organisms as stated above, i.e. non-pathogenic or weakly pathogenic, live enteric bacteria capable of eliciting an intestinal mucosa immune response to S. dysenteriae 1 O-antigen, optionally in combination with a pharmaceutically acceptable diluent and/or carrier.

Brief description of the drawing

Fig. 1 is a schematic diagram showing the construction of the plasmid pSS37.

Isolation of an R'-plasmid carrying the chromosomal O-polysaccharide biosynthesis genes of Shigella dysenteriae 1

To facilitate identification and isolation of chromosomal determinants required for S. dysenteriae 1 O-antigen production in E. coli K-12, R'-plasmids were generated that in combination with pSS8, a Tn5-tagged derivative of plasmid pHW400, provoke the formation of Shigella-LPS in this host. The RP4::miniMu plasmid pULB113 (van Gijsegem and Toussaint, Plasmid 7(1982)30-44), which specifies resistance to ampicillin, kanamycin and tetracycline, was transferred by conjugation into Shigella dysenteriae 1 strain W30864-22 (Watanabe and Timmis, Infect. Immun. 43(1984)391-396), which is resistant to streptomycin, kanamycin and ampicillin,and transconjugants selected on medium containing streptomycin, kanamycin and ampicillin. RP4::miniMu R'-plasmids containing the his gene region of S. dysenteriae 1 were then selected by transfer of RP4::miniMu from W30864-22 into the E. coli K-12 strain OT97 (pSS8) recA his Rif$^r$ with selection for histidine prototrophy and resistance to ampicillin, kanamycin, tetracycline and rifampicin. In order to identify those E. coli K-12 clones harbouring R'-plasmids carrying the Shigella rfb-gene-cluster, histidine prototrophic transconjugants were scored for expression of "S"-form (O-antigen containing) LPS.

Preliminary screening was carried out by scoring for resistance to the rough-specific bacteriophage T3 to which OT97 (pSS8) is sensitive. Several such transconjugants were identified and one, designated R' 40, was studied further. Lipopolysaccharide was isolated from this transconjugant by means of the phenol-water procedure of Westphal and Jann. (Meth. Carbon. Chem. 5 (1965), 83-91). Characterization of the LPS by polyacrylamide gel electrophoresis and immunoblotting with a polyclonal S. dysenteriae 1 antiserum demonstrated that it was indistinguishable from smooth LPS isolated from S. dysenteriae 1. R' 40 thus contains all information necessary for O-antigen production in E. coli K-12 OT97 (pSS8).

Construction of plasmid pSS3

The rfp gene was previously localized to a 2kb region of S.dysenteriae 1 plasmid pHW400 (Watanabe et al., Infect. Immun. 46(1984)55-63). In order to combine this gene with the cloned rfb gene cluster, a 4.7 kb EcoRV-ClaI fragment was first excised from pSS8 and ligated into pACYC184 plasmid DNA which had been digested with endonucleases EcoRV and ClaI (Fig. 1). The hybrid plasmid thereby generated was designated pSS3. When introduced into the pHW400 plasmid-lacking derivative of S. dysenteriae 1, strain W30864-22, it caused a resumption of production of O-antigen LPS. pSS3 thus carries and expresses the rfp gene. Plasmid pSS3 was deposited with the DSM under the deposition number DSM 3760.

Construction of plasmid pSS9

Cleavage of R'40 plasmid DNA with restriction endonuclease PstI generated 8 fragments which originated from vector pULB113, and 5 fragments with molecular weights of 11,5 kb, 11 kb (2x), 7,3kb and 4,8 kb which originated fromt he inserted Shigella chromosomal DNA. R' 40 plasmid thus contains an insert of about 46 kb. In order to clone the LPS genes carried by R' 40, R' 40 plasmid DNA was digested with PstI and the fragments thereby generated were ligated into PstI-cleaved pBR322 vector DNA. The ligation mixture was used to transform E. coli K-12 OT99 bacteria containing plasmid pSS3.

Ampicillin sensitive, tetracycline resistant transformants were screened for resistance to bacteriophage T3, and lipopolysaccharide isolated from two such clones. SDS-polyacrylamide gel electrophoresis of the LPS preparations and immunoblotting with polyclonal S. dysenteriae 1 antiserum confirmed that the clones expressed S. dysenteriae 1 0-polysaccharide. Digestion of plasmid DNA isolated from the two clones with endonuclease PstI revealed that both contained in addition to pSS3, pBR322 hybrid plasmids containing the 11.5 kb fragment of R' 40. One of the pBR322 hybrids, designated pSS9 was selected for further study. This plasmid was deposited with the DSM under the deposition number DSM 3761. Said recombinant

plasmid or the inserted DNA fragment can be used for the synthesis of the chromosomal enzymes involved in the biosynthesis of the O-antigen of Shigella dysenteriae 1. It can also be used as a probe for screening for nucleotide sequences coding for the chromosomal enzymes involved in the biosynthesis of the O-antigen of Shigella dysenteriae 1.

In order to analyze the organization of the cloned rfb gene cluster, and to identify the LPS biosynthesis functions encoded therein, a series of transposon Tn1000 insertion mutants of pSS9 were generated by F-mediated conjugal transfer. The E. coli K-12 C600 recA derivative used as a recipient in this cross , an OT99 derivative carrying pSS3, contained the rfp gene. On the basis of the sensitivity/resistance to rough-specific bacteriophage T3 of the transconjugants, of the banding patterns and immunoreactivity of LPS isolated from the transconjugants and electrophoresed on SDS-polyacrylamide gels, and of the sugar composition of the polysaccharide portion of the LPS determined by chemical analysis, six determinants for O-antigen production were identified and localized. Whereas E. coli K-12 LPS consists only of lipid A and core oligosaccharide, pSS3 containing derivatives additionally contained core oligosaccharide substituted with a galactose residue. At least two S. dysenteriae 1 chromosomal rfb determinants are involved in synthesis of TDP-rhamnose and the transfer of a rhamnose residue to the galactose-substituted core. One of these functions is probably TDP-rhamnose synthetase. A third function effects the transfer of a second rhamnose residue to the rha→gal-substituted core. A fourth function, for which evidence was obtained for two determinants (cistrons), is N-acetylglucosamine transferase, whereas a sixth determinant is necessary for extension of the first complete side chain repeat unit to the full O-antigen polymer. These results confirmed the previously-determined chemical composition of the S. dysenteriae 1 O-antigen and demonstrated that the order of the sugars is
glcNAc→rha→rha→gal→core
with gal as the first sugar linked to the core.

Construction of plasmid pSS37 (DSM 3762)

Plasmid pSS9 was introduced into E. coli strain OT99 (i.e. lacking pSS3) by transformation and a transformant used for large scale preparation of plasmid DNA. This was subjected to restriction endonuclease cleavage analysis, using a variety of enzymes singly and in appropriate combinations, and an endonuclease cleavage map constructed. As a result of this, a 9.2 kb EcoRV-EcoRI DNA fragment containing most of the S. dysenteriae 1 DNA insert in pSS9 was generated, isolated and ligated into EcoRV-EcoRI - digested pBR322 DNA and transformed into E. coli K-12 strain OT99 (pSS3). One hybrid plasmid thereby constructed was designated pSS23 (Fig. 1). SDS-polyacrylamide gel electrophoresis and immunoblotting revealed that the E. coli strain OT99(pSS3, pSS23) expresses S. dysenteriae 1 O-polysaccharide. The chromosomal LPS biosynthesis genes are thus contained within the 9.2 kb EcoRV-EcoRI fragment of plasmid pSS9. An 8.9 kb EcoRV-PvuII fragment of pSS9 was subsequently generated and separated on and isolated from a preparative agarose gel, and ligated into the unique EcoRV cleavage site of pSS3 (Fig. 1). The hybrid plasmid thereby formed, designated pSS37, when introduced into E. coli K-12 strain OT99 provoked the production of S. dysenteriae 1 O-specific lipopolysaccharide . It therefore constitutes a single plasmid containing all S. dysenteriae 1 genes needed to direct the production of S. dysenteriae 1 O-antigen in E. coli K-12, and presumably in other strains of bacteria.

Example 1

A) Isolation of an R'-plasmid carrying the chromosomal O-polysaccharide biosynthesis genes of Shigella dysenteriae 1

RP4:miniMu was transferred from MXR(pULB113) into S. dysenteriae 1 strain W30864-22 as follows. Donor and recipient strains were grown with gentle shaking in L-broth (LB) at 37°C to an absorbance $A_{600}$ of about 0.8. Portions of the cultures were then mixed and the mixture, plus portions of the original cutures, were concentrated 20-fold by centrifugation. 20-µl vols. of these suspensions were then placed on Antiobiotic Medium No. 3 (AM3; Difco) agar plates which were incubated for 2 h at 37°C. The bacterial growth from each drop was then resuspended in phosphate-buffered saline (PBS), and the suspensions were streaked onto AM3 agar plates containing kanamycin plus chloramphenicol. (Antibiotics were incorporated into media at the following concentrations: kanamycin, 50µg/ml; chloramphenicol, 50 µg/ml.) Transconjugants were identified by their resistance to these antibiotics and their agglutination in S.

dysenteriae 1-specific antiserum (Behringwerke, Marburg).

RP4: miniMu R'plasmids carrying segments of the S. dysenteriae 1 chromosome were isolated as follows. The donor strain was grown in LB at 32°C with gentle shaking until the culture reached an $A_{600}$ of 0.5 after which it was incubated at 43°C for 2 h to induce Mu functions. The recipient strain was grown in LB at 37°C for the same period of time. The matings were then carried out as described above, except that the period of mating was 3-6 h. After suspension of bacterial growths in PBS, cells were washed three times with PBS by centrifugation and resuspension, before plating on selective media.

B) Sensitivity of E.coli K-12 strains to rough-specific bacteriophage T3.

Bacteriophage T3 is a rough-specific LPS-phage which recognizes receptor structures in the core oligosaccharide of LPS. Bacteria producing high molecular weight O-polysaccharide, in which the core is substituted with the O side chain repeat unit, are resistant to T3 due to the fact that receptor structures of the core are blocked or masked.

Samples (0.1 ml) of appropriate phage dilutions were mixed with 0.1 ml of bacterial culture (grown to mid-exponential phase), incubated at 37°C for 20 min (preadsorption), mixed with 4 ml of soft agar, and overlaid on solid L-agar plates. These were incubated overnight at 37°C before being examined for phage plaques in the bacterial lawn.

C) Gel electrophoresis of LPS and immunoblotting.

Lipopolysaccharide was isolated from bacterial strains according to the phenol-water procedure of Westphal and Jann. SDS-polyacrylamide gel electrophoresis of purified LPS was performed as described by Laemmli (Nature 227 (1970)), 680-685) on 14 % polyacrylamide gels containing 4M urea. LPS bands were either visualized by staining with a silver stain (Biorad; or prepared by the method of Tsai and Frasch (Anal. Biochem. 119 (1982), 115-119), or transferred by electroblotting to nitrocellulose and detected with a polyclonal S. dysenteriae 1 antiserum as described by Sturm et al. (Arch. Microbiol. 140 (1984) 198-201).

Example 2

Construction of plasmid pSS3 (DSM 3760)

Plasmid pSS8 is a Tn5 transposon-containing derivative of the rfp gene-carrying plasmid pHW400 of Shigella dysenteriae 1 strain W30864. To 1µg of pSS8 DNA in 25µl of restriction buffer (10mM Tris-HCl, pH 7.5 - 10mM $MgCl_2$ -1mM dithiothreitol - 50mM NaCl) was added 2 units each of EcoRV and ClaI endonuclease and the solution was incubated at 37°C for 1 hr. 1µg of cloning vector pACYC184 DNA was similarly cleaved with EcoRV and ClaI in a separate reaction. The two solutions were then incubated at 65°C for 15 min. to inactivate the restriction endonucleases. The DNA in each solution was then precipitated with ethanol and subsequently resuspended in ligation buffer (50mM Tris-HCl, pH 7.5 - 10mM $MgCl_2$ -10mM dithiotreitol - 2mM ATP). The two DNA solutions were then mixed, 2 units of T4 DNA ligase added, and the mixture incubated at 15°C overnight. It was then used to transform competent cells of E.coli K-12 OT99 as described by Mandal and Higa (1970, J. Mol. Biol. 53, 159).

Minipreparations of plasmid DNA (Holmes & Quigley, 1981, Anal. Bioch. 114, 193) from chloramphenicol-resistant, tetracycline-sensitive transformant clones were analysed by cleavage with EcoRV + Cla1 and agarose gel electrophoresis, and clones containing hybrid molecules consisting of pACYC184 plus the 4.7 kb EcoRV - Cla1 fragment of pSS8 were retained. One such hybrid plasmid, which restored the ability of a pHW400 plasmid-lacking S. dysenteriae 1 isolate (W30864-22; Watanabe and Timmis, Infect. Immun. 43, (1984),391) to synthesise S. dysenteriae 1 O-antigen, was designated pSS3.

Example 3

A) Construction of plasmid pSS9 (DSM 3761)

To 2µg of plasmid R' 40 DNA in 25 µl of restriction buffer was added 4 units of PstI endoncuclease and the solution was incubated for 37°C for 1 hr. It was then incubated at 65°C for 15 min., mixed with 3µl of loading solution (0.25 % bromophenol blue - 15 % Ficoll type 400 - 0.1M EDTA in water) and applied to an agarose gel (0.6 % in 90mM Tris - 90 mM boric acid - 2.5 mM EDTA). Following electrophoresis at 20V overnight, the gel was stained for 30 min with ethidium bromide (0.5 µg/ml in water) and illuminated with long wave U.V. At least 13 fragments were visualized, five of which having molecular sizes of 4.8 kb, 7.3 kb, 11.0 kb (2 fragments) and 11.5 kb, originated from the S. dysenteriae 1 chromosome. A gel slice containing the 11.0 kb and 11.5 kb fragments was excised, placed in a dialysis bag, and the DNA recovered by electroelution as described by Maniatis et al. (Molecular Cloning - A Laboratory Manual, 1982, Cold Spring Harbor). These PstI-generated fragments of R' 40 were then ligated into PstI-cleaved pBR322 vector DNA and the hybrid plasmids thereby formed were transformed into competent E.coli K-12 OT99 (pSS3) bacteria. Tetracycline-resistant, ampicillin-sensitive transformants were screened for resistance to bacteriophage T3. Lipopolysaccharide was isolated from T3-resistant clones and analysed by SDS-polyacrylamide gel eletrophoresis and immunoblotting with polyclonal S. dysenteria 1 antiserum. Hybrid pBR322 plasmids present in E.coli K12 transformants which synthesized S.dysenteriae 1 O-antigen, and therefore contained not only the rfp gene of pSS3 but also the rfb genes derived from the S. dysenteriae 1 chromosome, were shown to contain the 11.5 kb PstI fragment of R' 40. One such hybrid plasmid, which contained only this 11.5 kb fragment, was designated pSS9.

B) Generation of Tn1000 (gamma-delta) insertion mutant derivatives of pSS9

E. coli K-12 donor strain TH 612 (F131, pSS9) was mated in liquid culture with recipient strain OT99 (pSS3) using the folliwng procedure: the donor was grown without shaking and 0.1 ml of a mid-exponential phase culture was mixed with 0.1 ml of an overnight culture of the recipient. 1 ml of L-broth was added and the conjugation mixture incubated at 37°C for 2 h without shaking. 0.1 ml aliquots were spread on AM3 plates containing tetracycline and chloramphenicol. Tetracycline and chloramphenicol resistant transconjugants were single colony isolated twice and screened for expression of O-specific polysaccharide with the phage T3 (see Ex. 1).

Transposon Tn1000 contains one ClaI site, which is 3kb from one end, and two HindIII and two EcoRI sites, see Guyer, J.Mol. Bid. 126(1978)347-363. The distance from the ends of the transposon is 1.1kb and 2.1kb for the HindIII sites and 0.9kb and 4kb for the EcoRI sites. The sites of insertions in pSS9::Tn1000 derivatives were localized relative to these endonuclease cleavage sites.

Example 4

Construction of plasmid pSS37 (DSM 3762)

To 2µg of pSS9 DNA in restriction buffer was added 4 units each of EcoRV and PvuII and the solution was incubated at 37°C for 1 hour. It was then incubated at 65°C for 15 min., mixed with 3yl of loading solution and applied to a 0.6 % agarose gel. Following electrophoresis at 20V overnight, the gel was stained with ethidium bromide and illumiated with long wave U.V. A gel slice containing an 8.9 kb EcoRV - PvuII fragment was excised and its DNA recovered by electrolution. The fragment thereby obtained with then ligated into EcoRV-cleaved pSS3 plasmid DNA and the ligation solution used to transform competent cells of E.coli K-12 OT99. Transformant clones that were resistant to bacteriophage T3 were shown to synthesis LPS that was electrophoretically indistinguishable from that of S. dysenteriae 1. The plasmid present in one such clone was designated pSS37.

## Claims

1. A DNA fragment of the chromosome of Shigella dysenteriae 1 characterized in that it contains the rfb-gene region of Shigella dysenteriae 1 which encodes the chromosomal nucleotide sugar synthetases and glycosyltransferases involved in the biosynthesis of the O-antigen of Shigella dysenteriae 1 having a length of 8.9 kb and having the following restriction map:

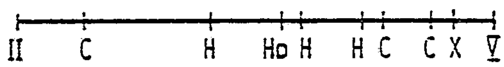


in which the following abbreviations are used: II, PvuII; C, Cla I; H, HindIII; Hp, HpaI; X, Xho I; V, EcoRV.

2. A DNA fragment hybridizing to a DNA fragment according to claim 1 and encoding the chromosomal nucleotide sugar synthetases and glycosyltransferases involved in the biosynthesis of the O-antigen of Shigella dysenteriae 1 or a part of the O-antigen, or an antigen that provokes formation of O-antigen cross-reacting antibodies.

3. A recombinant DNA molecule, characterized in that is comprises a DNA fragment according to claim 1 or 2.

4. A recombinant DNA molecule characterized in that it contains a DNA fragment according to claim 1 or 2 and a DNA fragment containing the genetic information for the plasmid-encoded Shigella dysenteriae 1 O-antigen biosynthesis function or functions.

5. A recombinant DNA molecule according to claim 4, charactterized in that the two DNA fragments are operatively linked to an expression control sequence.

6. The recombinant DNA molecule according to claim 5, characterized in that the expression control sequence is selected from the group of the E. coli promoter system, the E. coli lac system, the E. coli ß-lactamase system, the E. coli trp-system and the E. coli lipoprotein promoter.

7. The recombinant DNA molecule according to claim 4 or 5, characterized in that the DNA fragment containing the genetic information for the plasmid encoded Shigella dysenteriae 1 O-antigen biosynthesis function(s) is the rfp-gene region of plasmid pHW400 of Shigella dysenteriae 1.

8. The recombinant DNA molecule according to claim 7,characterized in that it is plasmid pSS37, deposited with the DSM under the deposition number DSM 3762.

9. A host organism, characterized in that it is a gram-negative bacterium which is transformed with a recombinant DNA molecule according to any one of claims 4 to 8.

10. The host organism according to claim 9, characterized in that it is a species of the genera Shigella, Salmonella or Escherichia.

11. The host organism according to claim 9, characterized in that it is the species Escherichia coli K12.

12. Th host organism according to claim 11, characterized in that it is capable of invading the human intestinal epithelium.

13. A process for preparing Shigella dysenteriae 1 O-antigen producing bacteria, characterized by cultivating a transformed host according to anyone of claims 9 to 12 in a suitable medium, and recovering said bacteria.

14. A vaccine for preventing bacillary dysentery, characterized in that it contains a transformed non-pathogenic or weakly pathogenic host in combination with a pharmaceutically acceptable diluent and/or carrier.

15. A vaccine for preventing bacillary dysentery, comprising Shigella dysenteriae 1 O-antigen-containing material encoded by a DNA fragment according to claim 1 or 2 in combination with a pharmaceutically acceptable diluent and/or carrier.

16. A method of preventing bacillary dysentery, comprising administering enterally to human being a vaccine according to claim 14 in an amount sufficient to induce an immuno-response or to modulate immuno-reactions.

17. A method of preventing bacillary dysentery, comprising administering enterally to a human being a vaccine according to claim 15.

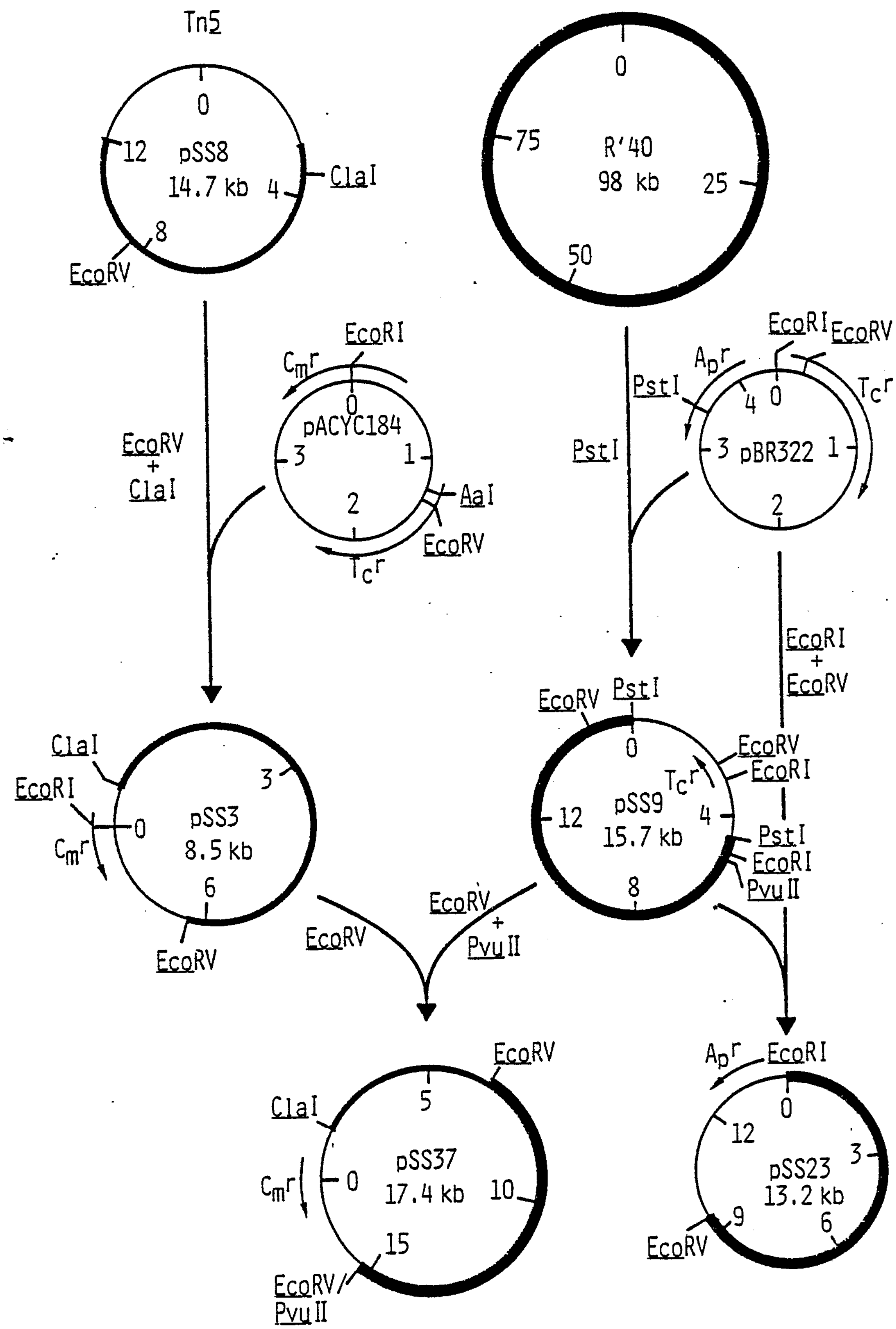
Tn5
0
12
pSS8
14.7 kb
4
ClaI
8
EcoRV
0
75
R'40
98 kb
25
50
EcoRV
+
ClaI
EcoRI
Cmr
0
pACYC184
3
1
2
AaI
EcoRV
Tcr
PstI
PstI
EcoRI
EcoRV
Apr
4
0
Tcr
3
pBR322
1
2
EcoRI
+
EcoRV
ClaI
EcoRI
3
0
Cmr
pSS3
8.5 kb
6
EcoRV
EcoRV
PstI
0
EcoRV
EcoRI
Tcr
12
pSS9
15.7 kb
4
PstI
EcoRI
PvuII
8
EcoRV
EcoRV
+
PvuII
EcoRV
ClaI
5
Cmr
0
pSS37
17.4 kb
10
15
EcoRV/
PvuII
Apr
EcoRI
0
12
3
pSS23
13.2 kb
9
6
EcoRV

European Patent Office

**PARTIAL EUROPEAN SEARCH REPORT**
which under Rule 45 of the European Patent Convention shall be considered, for the purposes of subsequent proceedings, as the European search report

Application number
EP 86 10 8541

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X<br><br>Y | INFECTION AND IMMUNITY, vol. 46, no. 2, November 1984, pages 470-475 Am. Soc. for Microbiology, US T.L. HALE et al.: "Expression of lipopolysaccharide O antigen in Escherichia coli K-12 hybrids containing plasmid and chromosomal genes from Shigella dysenteriae 1."<br>* Whole article *<br>--- | <br><br>1-3<br>4-13 | C 12 N 15/00<br>C 12 N 1/20<br>A 61 K 39/112<br>A 61 K 39/108//<br>(C 12 N 1/20<br>C 12 R 1:01<br>1:42<br>1:185) |
| Y,D | INFECTION AND IMMUNITY, vol. 43, no. 1, January 1984, pages 391-396; Am. Soc. for Microbiology, US H. WATANABE et al.: "A small plasmid in Shigella dysenteriae 1 specifies one or more functions essential for O antigen production and bacterial virulence."<br>* Whole article *<br>---<br>./. | 4-13 | **TECHNICAL FIELDS SEARCHED (Int. Cl.4)**<br>C 12 N<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-15
Claims searched incompletely:
Claims not searched: 16,17
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 24.02.1987 | CUPIDO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number EP 86 10 8541



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | EP-A-0 080 806 (RESEARCH CORP.)<br>* Claims 1-10 * | 14,15 | |
| | | | TECHNICAL FIELDS SEARCHED (Int. Cl.4) |